(19) Europäisches Patentamt / European Patent Office / Office européen des brevets

(11) **EP 4 188 328 B1**

(12) **EUROPEAN PATENT SPECIFICATION**

(45) Date of publication and mention
of the grant of the patent:
**15.07.2026 Bulletin 2026/29**

(21) Application number: **21762346.1**

(22) Date of filing: **27.07.2021**

(51) International Patent Classification (IPC):
***A61K 9/00*** *(2006.01)*     ***A61K 9/16*** *(2006.01)*
***A61K 38/00*** *(2006.01)*

(52) Cooperative Patent Classification (CPC):
**A61K 9/1658; A61K 9/0053; A61K 9/1694;**
A61K 38/00

(86) International application number:
**PCT/EP2021/071088**

(87) International publication number:
**WO 2022/023392 (03.02.2022 Gazette 2022/05)**

(54) **A PHARMACEUTICAL COMPOSITION IN ORAL DOSAGE FORM**

PHARMAZEUTISCHE ZUSAMMENSETZUNG IN ORALER DARREICHUNGSFORM

COMPOSITION PHARMACEUTIQUE SE PRÉSENTANT SOUS LA FORME D'UNE FORME
POSOLOGIQUE ORALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.07.2020 GB 202011598**

(43) Date of publication of application:
**07.06.2023 Bulletin 2023/23**

(73) Proprietor: **Teagasc - The Agriculture and Food
Development
Authority
Carlow, Co. Carlow (IE)**

(72) Inventors:
• **BLEIEL, Sinead**
Dublin, D2 (IE)
• **SEVERIC, Maja**
Cork T12 TX06 (IE)

(74) Representative: **Purdylucey Intellectual Property**
**23 Ely Place**
**Dublin 2 D02 N285 (IE)**

(56) References cited:
• **DÉAT-LAINÉ EMMANUELLE ET AL:
"Development and in vitro characterization of
insulin loaded whey protein and alginate
microparticles", INTERNATIONAL JOURNAL OF
PHARMACEUTICS, vol. 439, no. 1-2, 1 December
2012 (2012-12-01), NL, pages 136 - 144,
XP055789448, ISSN: 0378-5173, DOI: 10.1016/
j.ijpharm.2012.10.003**
• **EMMANUELLE D?AT-LAIN? ET AL: "Efficacy of
Mucoadhesive Hydrogel Microparticles of Whey
Protein and Alginate for Oral Insulin Delivery",
PHARMACEUTICAL RESEARCH, vol. 30, no. 3,
24 October 2012 (2012-10-24), pages 721 - 734,
XP055162210, ISSN: 0724-8741, DOI: 10.1007/
s11095-012-0913-3**
• **DÉAT-LAINÉ EMMANUELLE ET AL: "Whey
protein and alginate hydrogel microparticles for
insulin intestinal absorption: Evaluation of
permeability enhancement properties on Caco-2
cells", INTERNATIONAL JOURNAL OF
PHARMACEUTICS, ELSEVIER, NL, vol. 453, no.
2, 21 June 2013 (2013-06-21), pages 336 - 342,
XP028682697, ISSN: 0378-5173, DOI: 10.1016/
J.IJPHARM.2013.06.016**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the Invention

The present invention relates to a pharmaceutical composition in an oral dosage form, as defined by the claims, suitable for delivery of poorly permeable drugs such as peptides into the bloodstream of a subject via an oral route.

Background to the Invention

Peptides are single amino acids (monomers) attached by peptide bonds that are up to 50 amino acids in length (<30kDa). They sit between small molecules (<0.9 kDa Daltons) and biopharmaceutical drugs (biologics). Peptides bind to cells and communicate instructions (transduce signals) to the cells that modify the cells' behaviour. Peptides can be used therapeutically as peptide hormones for example to replicate the functionality of erythropoietin (EPO) and Insulin where they typically target a cell receptor and elicit a pharmacological response. Peptides are cheaper to produce than biologics but suffer from weaknesses such as instability, short half-lives and bioavailability when in the body. These weaknesses can be negated by modification of the peptides or use of permeation enhancers but these modifications may decrease the in vivo efficacy. Therefore, most therapeutic peptides (75%) on the market are injected directly into the bloodstream (parenteral) as opposed to via the skin (topical or transdermal) or through the gastrointestinal tract (enteral). There are now several orally available peptide drugs on the market and in clinical development. However, Interferon for example (20kDa), is too large to cross the gut wall meaning orally delivered therapeutic peptides have to be sized between 0.9 kDa and 20 kDa.

Most peptide therapeutics have low intestinal permeability, requiring the peptide to be overdosed, adding to the costs of the therapeutic. In addition, due to poor permeability, many oral peptide therapeutics include permeation enhancer molecules that assist in the transport of poorly absorbed peptides across the intestinal epithelium. Examples of permeation enhancers in development include toxins and their derivatives, and surfactant-based permeation enhancers. A problem with many of these chemicals is that they cause damage to the gut wall and in many cases pain to the subject.

Insulin-loaded whey protein microcapsules are described in Deat-Laine et al (International Journal of Pharmaceutics, Vol. 239, 2012; Pharmaceutical Research, Vol. 30, 20212; and International Journal of Pharmaceutics, Vol. 453, 2013). All of these microcapsules have a matrix formed of whey protein and alginate and provide microcapsules with good mucoadhesive properties but unstable gelation that favours early insulin release in the stomach. However, the microparticles are not suitable for in-vivo delivery and have to be administered inside an enteric capsule which reduces mucoadhesive properties resulting in sub-optimal intestinal uptake and complicates manufacture. EMMANUELLE DÉAT-LAINÉ ET AL: "Efficacy of Mucoadhesive Hydrogel Microparticles of Whey Protein and Alginate for Oral Insulin Delivery", PHARMACEUTICAL RESEARCH, vol. 30, no. 3, 24 October 2012 (2012-10-24), pages 721-734: Insulin transport via duodenal membranes by relevant microparticles of insulin and denatured whey protein isolate (WPI)/alginate prepared by cold gelation and a CaCl2 bath and subsequent freeze drying (under vacuum).

It is an object of the invention to overcome at least one of the above-referenced problems.

Summary of the Invention

The Applicant has addressed the problems of the prior art by providing an oral dosage form that can be used to deliver poorly permeable drugs such as peptides via the oral route, without the need for high levels of over-dosing, without the need to be packaged inside an enteric capsule, and without using damaging permeation enhancer chemicals. The oral dosage form comprises microcapsules having a protein matrix and insulin contained and protected within the matrix, as defined by the claims. The protein is whey protein, as defined by the claims.

Further disclosed, but not claimed, is also another protein containing ß-lactoglobulin. The matrix is resistant to gastric conditions and configured to break up in the ileum, releasing the contents gradually over a period of time. In addition, the Applicant has also discovered that ß-lactoglobulin, a protein component of whey protein, assists with transport of poorly permeable drugs across the intestinal epithelium, obviating the need for gut wall damaging permeation enhancers in the formulation. As an example, the Applicant shows that an oral dosage form comprising microcapsules having a whey protein isolate matrix and insulin aspart API contained within the matrix (25-100 IU insulin per dose) can be successfully delivered via the oral route in a subject with Type I diabetes, resulting in a 25-42% reduction in blood glucose over a 3 hour period.

The Applicant has also discovered that microcapsules produced by cold gelation (e.g. extrusion into a curing/gelation bath), and drying by vacuum drying, are ideal for delivering heat-sensitive active agent such as peptides via the oral route, as the cold gelation and vacuum drying processes avoid the conventional high heat processes conventionally used for producing microparticles, resulting in less thermal deactivation of the peptide and greater payload of active peptide. In addition, as the matrix does not require an additional swelling / gelation agent such as alginate, and as such contains less water after cold gelation compared with the microcapsules of the prior art that contain whey protein and alginate, the level of drying required to achieve a desired level of water activity is lower. In one aspect, the microcapsules are vacuum dried in two vacuum drying steps, providing dried microcapsules that are resistant to moisture and heat.

The invention provides an oral dosage form comprising microcapsules, as defined by the claims, comprising a matrix and an active agent contained within the matrix, in which the matrix comprises denatured protein.

The microcapsules are cold-gelated and vacuum dried.

In any embodiment, at least 50%, 60%, 70%, 80%, 90%, 95% or 99% by weight of the matrix is protein.

The microcapsules are substantially free of a polysaccharide based gelling agent such as alginate.

The active agent is a poorly permeable active pharmaceutical ingredient (API), which is insulin as defined by the claims. Insulin may be insulin degludec or insulin aspart.

In any embodiment, the pharmaceutical composition is provided as a unit dose comprising 10-100 IU of insulin.

In any embodiment, the unit dose comprises 10-50 IU of insulin.

In any embodiment, at least 90% of the insulin in the oral dosage form is active.

In any embodiment, the oral dosage form is free of permeation enhancer, such as chitosan or surfactant-type permeation enhancer.

In any embodiment, the whey protein is provided by whey protein isolate, or whey protein concentrate.

In any embodiment, at least 50%, 60%, 70%, 80%, 90% or 95% (by weight) of the oral dosage form is constituted by microcapsules.

The denatured whey protein is gelated.

The microcapsules are formed by extrusion and gelation/ polymerisation in a gelation bath.

The microcapsules are vacuum dried.

In any embodiment, the microcapsules are provided as agglomerates of microcapsules. This can be achieved by varying the drying conditions to promote agglomeration during drying. An advantage of agglomerated microcapsules is that they delay the release of active agent contained within the matrix of the individual microcapsules. Once agglomerates reach the ileum, the agglomerate must first break up to release individual microcapsules, before the released micro-capsules are broken down to release the encapsulated active agent. This results in a prolonged release profile, which can be tailored by modifying the drying conditions of the microcapsules. Agglomerates of microcapsules are ideal for delivering active agents into the ileum for which a slow prolonged release is desirable.

Drying can be tailored to generated agglomerates using conditions such as vacuum pressure, temperature, agitation, and fill volume. The drying time can be completed within 30 minutes to 12 hours. Drying occurs using vacuum.

In any embodiment, the microcapsules comprise:

40-80 % denatured whey protein (w/w); and
20-60% insulin (w/w).

In any embodiment, the microcapsules comprise:

60-75 % denatured whey protein (w/w); and
15-40% insulin (w/w).

In any embodiment, the microcapsules comprise as a dry weight %:

90 to 99 % denatured whey protein (w/w); and
1 to 10 % insulin (w/w).

Dry weight % means the % of the microcapsule in a dry form.

In any embodiment, the microcapsules or agglomerates of microcapsules have an average particle size of 200 to 1000 microns.

In any embodiment, the microcapsules or agglomerates of microcapsules have an average particle size of 200 to 500 or 300 to 500 microns.

In any embodiment, the microcapsules or agglomerates of microcapsules have an average particle size of 500 to 1000 microns.

In any embodiment, the microcapsules or oral dosage form is free of one or all of chitosan, toxin permeation enhancers, surfactant-type permeation enhancer, and cell penetrating peptides. The denatured whey protein acts as a permeation enhancer obviating the need for additional permeation enhancers.

In any embodiment, the oral dosage form is a solid. Examples include tablets, capsules, granules, flakes and powders.

In any embodiment, the oral dosage form is a semi-solid, such as a gel.

In any embodiment the oral dosage form is a liquid, such as a suspension.

In any embodiment, the oral dosage form comprises a pharmaceutical excipient.

In any embodiment, the oral dosage form comprises a second active agent.

Typically, the whey protein matrix comprises at least 30%, 40%, 50%, 60%, 70%, or 80% β-lactoglobulin (w/w). Suitably, the β-lactoglobulin has a degree of denaturation of at least 50%, 60%, 70%, 80%, 90% or 95%. In a preferred embodiment of the invention, the whey protein comprises at least 70% β-lactoglobulin having a degree of denaturation of at least 90%.

Typically, at least 90% of the microcapsules in the preparation are capable of surviving intact in fresh ex-vivo porcine gastric juice of pH 2.0 for at least three hours at 37°C under agitation at 150rpm (employing the methods as described below).

In another aspect, the invention provides an oral dosage form according to the invention, for use in a method of treating a disease or condition in a subject, in which the oral dosage form is administered orally to the subject.

In any embodiment, the active agent is a peptide API, and in which the disease or condition is a disease or condition indicated for the peptide API. Examples of peptide API's include insulin (diabetes), desmopressin (water diabetes), octreotide (acromegaly, carcinoid tumors), cyclosporin (organ rejection), vanomycin (antibiotic), semaglutide (type II diabetes), exenatide (type II diabetes), Liraglutide (obesity, type II diabetes), Orlistat (obesity, type II diabetes).

In any embodiment, the disease is a metabolic disease.

In any embodiment, the metabolic disease is diabetes, for example type I diabetes or type II diabetes.

In any embodiment, the condition is elevated blood sugar levels.

In any embodiment, the blood glucose levels of the subject are reduced by at least 10%, 15%, 20%, 25%, 30%, 35%, 40%, 45% or 50% within 4 or 5 hours of administration of the oral dosage form to the subject.

In any embodiment, the disease is obesity.

In any embodiment, the oral dosage form is administered to the subject once a day.

In another aspect, the invention provides a method of making microcapsules comprising the steps of

generating microdroplets comprising denatured protein and active agent by extrusion through a nozzle; curing the microdroplets by immersion in a curing bath to form microcapsules having a matrix comprising denatured whey protein and active agent contained within the matrix; removing the microcapsules from the curing bath; and drying the microcapsules.

In any embodiment, the microcapsules prior to drying comprise less than 70%, 60%, 55%, 50% or 45% water by weight (or a water activity (Aw) of 0.993 - 0.997).

The drying step comprises vacuum drying the microcapsules. In any embodiment, the drying step comprises fluidised bed drying the microcapsules.

In any embodiment, the drying conditions are configured to agglomerate the microcapsules during the drying step, and preferably provide agglomerates having an average dimension of 0.5 to 3, 0.1 to 0.3 or 1 to 2, microns. Drying conditions can be varied to change morphology of particles (i.e. discrete microcapsules or agglomerates of microcapsules). For example, the batch size of micro-capsule size will determine the time of drying and a combination of these factors will generate optimal microcapsules. The creation of agglomerates of microcapsules is in one embodiment defined by the flowrate, speed of agitation and batch size.

In any embodiment, the speed of agitation is the microcapsules during drying is 150rpm to 800rpm.

In any embodiment, the temperature during drying is 15°C- 60°C.

In any embodiment, the flow rate 10ml/min to 1.5L/min.

In any embodiment, the microcapsules are vacuum dried for 18-24 hours.

In any embodiment, the microcapsules are vacuum dried to a moisture content of less than 8%, 7%, 6% or 5% by weight.

In any embodiment, the microcapsules are vacuum dried to a water activity (Aw) of less than 0.25 or 0.20.

In any embodiment, the microcapsules are subjected to a first vacuum drying step and a second vacuum drying step. The reason for double drying is to provide better stability against moisture and heat. Double dried microcapsules can resist cell death during pasteurisation (78°C for 15 min) and UHT processes (143°C for 4.5 seconds). It is really important to be able to include moisture stability and thermal protection (heat stability) to the claims.

In any embodiment, the microcapsules are agitated during the first vacuum drying step.

In any embodiment, the microcapsules are agitated during the second vacuum drying step. In any embodiment, the microcapsules are not agitated during the second vacuum drying step.

In any embodiment, the second vacuum drying step is performed at a pressure lower than the pressure of the first vacuum drying step.

In any embodiment, the first vacuum drying step is performed at a pressure of less than 15 mBar (e.g. 5 or 10 -15 mBar).

In any embodiment, the second vacuum drying step is performed at a pressure of less than 10 mBar (e.g. 5-10 mBar).

In any embodiment, the microdroplets are generated using concentric nozzles by extruding a suspension or solution of the active agent optionally including suspended denatured protein (generally whey protein) through an inner nozzle and simultaneously extruding a denatured protein suspension through an outer nozzle. This step forms core-shell type microcapsules (e.g. mononuclear microcapsules). The protein suspension generally comprises 5 to 20, 5 to 15, 8 to 14, or 10-12 or 10 to 20 % denatured whey protein by weight.

In any embodiment, the microdroplets are generated by extruding a suspension comprising denatured protein (generally whey protein) and active agent through a single nozzle. This produces microcapsules with a continuous protein matrix and pockets of active agent distributed throughout the matrix (e.g. multinuclear microcapsules). The protein suspension generally comprises 5 to 20, 5 to 15, 8 to 14, or 10-12 or 10 to 20 % denatured whey protein by weight.

In any embodiment, the active agent is a peptide.

In any embodiment, the peptide is insulin or an insulin analogue.

In any embodiment, the protein suspension comprises whey protein, generally denatured whey protein.

In any embodiment, the step of providing the suspension comprises the steps of: dissolving insulin in an acid to provide an insulin solution;

> combining the insulin solution with a buffer to provide a buffered insulin solution with a pH to 6-8; and
> combining the buffered insulin solution with the suspension comprising denatured whey protein

In any embodiment, the buffered insulin solution is added to the suspension dropwise.

In any embodiment, the volume ratio of buffered insulin solution to protein suspension is 1:3 to 1: 60, for example 1:5 to :1:50, for example 1:3 to 1:7, 1:10 to 1:30, or 1: 40 to 1:60.

In any embodiment, the whey protein is selected from protein isolate (WPI) or whey protein concentrate (WPC).

In any embodiment, the buffered insulin solution comprises 0.1 to 1.0%, 0.2 to 0.6% and preferably 0.3 to 0.5% insulin (w/v).

In any embodiment, the method has an encapsulation efficiency of at least 90%, 95%, 98%, 99% or 99.5% as determined using the method described below.

In any embodiment, the buffered insulin solution comprises hydrochloric acid.

In any embodiment, the curing bath comprises a citrate buffer. In any embodiment, the curing bath is heated (for example 25-45°C). In any embodiment, the curing bath has a acidic pH, for example 3-5, 4-5, or 4.2 to 4.5.

In any embodiment, the microcapsules are allowed to polymerise in the curing bath for at least 30, 60, 90 ,120 or 140 minutes.

In any embodiment, the microcapsules are washed after polymerisation in a washing solution. In any embodiment, the washing solution is water, milk powder, or water and then milk powder (generally skim milk powder).

In another aspect, the invention provides a pharmaceutical composition in an oral dosage form, as defined by the claims, comprising a poorly permeable drug and a composition comprising β-lactoglobulin and optionally an acceptable pharmaceutical excipient. The poorly permeable drug is insulin. The ß-lactoglobulin may be denatured. The ß-lactoglobulin may be provided by whey protein, for example WPI or WPC. The oral dosage form may comprise microcapsules comprising a matrix comprising denatured whey protein and poorly permeable drug contained within the matrix, as defined by the claims, typically contained in pockets distributed throughout the matrix. The microcapsule may be formed by extrusion of microdroplets and curing in a curing bath. The matrix is gelated The matrix is gastric resistant and ileal sensitive. The microcapsules are vacuum dried.

In another aspect, the invention provides the use of a composition comprising ß-lactoglobulin as a permeation enhancer in a pharmaceutical composition. The pharmaceutical composition is generally an oral dosage pharmaceutical composition. Exemplary compositions comprising β-lactoglobulin include whey protein, especially denatured whey protein. The whey protein may be WPI or WPC. The composition comprising β-lactoglobulin may constitute 10-90%, 10-70%, 10-50%, 10-30%, 10-20%, 20-60%, 20-40%, 40-80%, 40-60%, 50-90%, 60-90%, 70-90%, 1-20%, 5-20%, 10-20%, or 80-90% by weight of the pharmaceutical composition.

Other aspects and preferred embodiments of the invention are defined and described in the other claims set out below.

Brief Description of the Figures

> **FIG. 1A** shows microscope image of vacuum dried insulin loaded microcapsules according to the invention.

**FIG. 1B** shows (human eye visual) homogenous size distribution of a batch of vacuum dried agglomerates of insulin loaded microcapsules according to the invention .

**FIG. 2A** shows (microscope image x40 ) even size distribution of vacuum dried agglomerates of insulin loaded microcapsules according to the invention .

**FIG. 2B** shows (microscope image x40) vacuum dried agglomerates of insulin loaded microcapsules according to the invention

**FIG. 2C** shows (Scanning Electron Microscope image 10um) of vacuum dried agglomerates of insulin loaded microcapsules according to the invention

**FIG. 3A** shows (Scanning Electron Microscope image 10um) of vacuum dried agglomerates of insulin loaded microcapsules according to the invention

**FIG. 3B** shows (Confocal Scanning Microscope image) of vacuum dried insulin loaded microcapsules (before agglomeration) according to the invention

**FIG. 4** shows agglomerates of fluidised -dried insulin loaded microcapsules according to the invention.

**FIG. 5** shows agglomerates of vacuum-dried insulin loaded microcapsules according to the invention.

**FIG. 6** shows agglomerates of vacuum-dried insulin loaded microcapsules according to the invention.

**FIG. 7** shows the effects of oral dosage of micro-encapsulated insulin (17.7 to 50 IU FIASP) on blood glucose levels (mmol/l) in a Type I diabetes patient. Figure7 represents *"Units of reduction after 3 h of dosage (expressed in **mmol glucose/L; red bold line ---**) and "% Reduction" (**white columns**) compared to subcutaneous injection of 5 IU of insulin (control; **black, initial column**). Ingested insulin microbeads (white columns) were consumed at different doses including 17.9, 25 and 50 units).*

**FIG. 8** shows the effects of oral dosage of micro-encapsulated insulin (50 IU FIASP) on blood glucose levels (mmol/l) in a Type I diabetes patient. Figures illustrates *"Units of reduction after 3 h of dosage (**mmol glucose/L; red line ---**) and "% Reduction" (**black columns**) after ingestion of 50 units of micro-encapsulated insulin*

**FIG. 9A and 9B.** Fourier-Transform IR spectrophotometric graph (Vector normalised at1,580-1,710cm$^{-1}$) for denatured protein and comparison with milk protein with various levels of denaturation.

**FIG.10.** Difference in the area under the curve of glucose concentration after ingestion of micro-encapsulated insulin (50 units).

**FIG 11.** Difference in the area under the curve of glucose concentration during and after injected of aspart insulin.

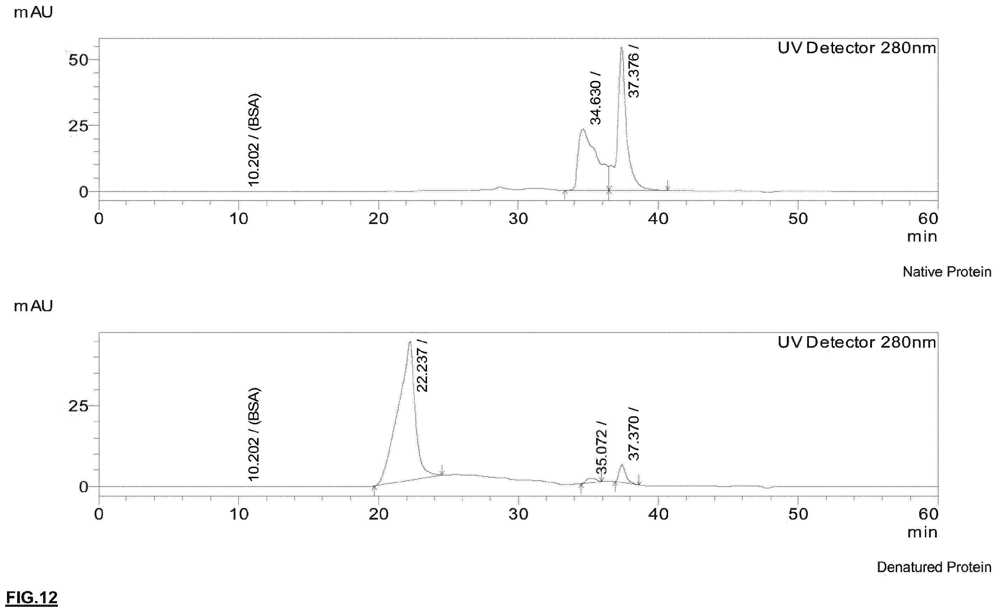

**FIG. 12.** HPLC chromatogram to exemplify protein denaturation optimal encapsulation

Detailed Description of the Invention

Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa*. The term "a" or "an" used in relation to an entity is to be read to refer to one or more of that entity. As such, the terms "a" (or "an"), "one or more," and "at least one" are used interchangeably herein.
As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae*, which includes horses, donkeys, asses, kiang and zebra.

As used herein, the term "oral dose form" refers to a pharmaceutical composition formulated for oral administration. Examples include tablets, pills, capsules, thin films, pastes, gels, powders, granules, liquid solutions or suspension. Tablets may be formed by direct compression. The oral dosage form generally includes an active agent and a pharmaceutical excipient. In one aspect of the present invention, the active agent is provided in the form of a microcapsule, in which the active agent is contained within a matrix configured to protect the active agent during gastric transit and release the active agent in the ileum. To this end, the matrix may comprise gelated denatured protein such as whey protein. In one embodiment the oral dosage form is provided as a unit dose, containing a single dose of active agent. In the case of diabetes for example, this may be 10-100 IU of insulin.

As used herein the term "poorly permeable active pharmaceutical ingredient" refers to an active biopharmaceutical ingredient that is classed as a Class 3 or 4 under the Biopharmaceutical Classification System (BCS) published by the FDA December 2017. Examples include peptide therapeutics such as insulin, desmopressin, octreotide, cyclosporin, vanomycin, salmon calcitonin, semaglutide, exenatide and insulin or an insulin analogue. Insulin may be insulin degludec or insulin aspart.

The term "microcapsule" as used herein should be understood to mean a particle comprising an active component encapsulated within a matrix comprising denatured whey protein. Preferably, the microcapsule has an average diameter (average particle size) of 200 to 1000 microns. Size is determined using a method of laser diffractometery (Mastersizer 2000, Stable Micro Systems, Surrey, UK). This method is determines the diameter, mean size distribution and D (v, 0.9) (size at which the cumulative volume reaches 90% of the total volume), of micro-encapsulates with diameters in the range of 0.2-2000 $\mu$m. For insulin microcapsule size analysis, micro-encapsulate batches were resuspended in Milli-Q water and size distribution is calculated based on the light intensity distribution data of scattered light. Measurement of microencapsulate size is performed at 25°C and six runs are performed for each replicate batch (Doherty et al., 2011) (Development and characterisation of whey protein micro-beads as potential matrices for probiotic protection,S.B. Doherty, V.L. Gee, R.P. Ross, C. Stanton, G.F. Fitzgerald, A. Brodkorb, Food Hydrocolloids Volume 25, Issue 6, August 2011, Pages 1604-1617). A preferred method of producing the microcapsules in by extrusion through a nozzle, typically a vibrating nozzle, and curing (gelation) in a gelation bath. In one embodiment, the suspension is sprayed through a nozzle and laminar break-up of the sprayed jet is induced by applying a sinusoidal frequency with defined amplitude to the spray nozzle. Examples of vibrating nozzle machines are and EnCapsulator (Inotech, Switzerland), or equivalent scale-up version such as those produced by Brace GmbH or Capsulae and the like. Typically, the spray nozzle has an aperture of between 100 and 600 microns, preferably between 150 and 400 microns, suitably about 300 microns. Suitably, the frequency of operation of the vibrating nozzle is from 900 to 4000 Hz. Generally, the electrostatic potential between nozzle and acidification bath is 0.85 to 1.8 V. Suitably, the amplitude is from 4.7kV to 7kV. Typically, the falling distance (from the nozzle to the curing bath) is less than 100cm, preferably less than 80cm, suitably between 50 and 70cm, preferably between 45 and 65cm, and ideally about 55 cm. The flow rate of suspension (passing through the nozzle) is typically from 3.0 to 120 ml/min; an ideal flow rate is dependent upon the nozzle size utilized within the process.

"Cold-gelated" as applied to microcapsules refers to microcapsules having a gelated protein matrix formed by extrusion through a nozzle and curing (gelation) is a gelation bath. Cold gelated microcapsules may be extruded through a single nozzle (in which the matrix contains pockets of active agent) or through a double concentric nozzle which form core-shell type microcapsules. Method of producing microcapsules by cold-gelation is described in WO2010/119041, WO2014/198787 and WO2016/096929.

"Gastro-resistant": means that the microencapsulates can survive intact for at least 60 minutes in the simulated stomach digestion model described in Minekus et al., 1999 and 2014 (A computer-controlled system to simulate conditions of the large intestine with peristaltic mixing, water absorption and absorption of fermentation product, Minekus, M., Smeets-Peeters M, Bernalier A, Marol-Bonnin S, Havenaar R, Marteau P, Alric M, Fonty G, Huis in't Veld JH, Applied Microbiology Biotechnology. 1999 Dec;53 (1):108-14) and (Minekus et al., 2014, A standardised static in vitro digestion method suitable for food - an international consensus, Minekus, A. et al., Food Function, 2014, 5, 1113).

"Ileal-sensitive": means that the microencapsulates are capable of releasing their contents in vivo in the ileum of a mammal.

"Encapsulation efficiency" means the amount of insulin loaded into the microcapsule carrier. The Encapsulation efficiency is calculated as follows by determining the free insulin concentration, and the total amount of insulin (Initial insulin concentration).

$$\text{EE \% = 100 - [(Free insulin conc. / Initial insuling conc.) x 100]}$$

"Vacuum drying" is the mass transfer operation in which the moisture present in a substance, usually a wet solid, is removed by means of creating a vacuum. In chemical processing industries like food processing, pharmacology, agriculture, and textiles, drying is an essential unit operation to remove moisture. Vacuum drying is generally used for the drying of substances which are hygroscopic and heat sensitive, and is based on the principle of creating a vacuum to decrease the chamber pressure below the vapor pressure of the water, causing it to boil. With the help of vacuum pumps, the pressure is reduced around the substance to be dried. This decreases the boiling point of water inside that product and thereby increases the rate of evaporation significantly. The result is a significantly increased drying rate of the product. The pressure maintained in vacuum drying is generally 0.03-0.06 atm and the boiling point of water is 25-30 °C. The vacuum drying process is a batch operation performed at reduced pressures and lower relative humidity compared to ambient pressure, enabling faster drying. "Water activity" (aw) is the partial vapor pressure of water in a substance divided by the standard state partial vapor pressure of water. It is measured by the method described in Carter, B. P., Galloway, M. T., Campbell, G. S., & Carter, A. H. (2015). The critical water activity from dynamic dewpoint isotherms as an indicator of pre-mix powder stability. Journal of Food Measurement and Characterization, 9(4), 479-486. The operator's manual of the equipment used is provided at http://manuals.decagon.com/Manuals/13893_AquaLab%20Pre_Web.pdf Values for water activity (Aw) provided herein are obtained at 25°C unless stated otherwise.

"Fluidised air drying" refers to an air-drying approach to control the gentle and homogenous drying of wet solids such as micro-capsules. The intensive mass exchange of air fluidized between micro-capsules makes this method effective and suitable for post-drying of micro-capsules and agglomerated micro-capsules.

The present invention also provides pharmaceutical compositions. Such compositions comprise an effective amount of microcapsules or agglomerates according to the invention, and a pharmaceutically acceptable excipient or carrier. In a specific embodiment, the term "pharmaceutically acceptable" means approved by a regulatory agency of the Federal or a state government or listed in the U.S. Pharmacopeia or other generally recognized pharmacopeia for use in animals and more particularly in humans. The term "excipient" refers to a diluent, adjuvant, excipient, or vehicle with which the microcapsules or agglomerates of the invention are administered. Such pharmaceutical excipients can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol and water.

Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

## Example 1 - Microcapsules

*Insulin buffer preparation.*

Weight 1 g of insulin into a weight boat.
Note: the amount of insulin and reagents will depend on the size of the batch needed and the availability of reagents.
Pipette 7.5 mL HCl (0.2M).
Add insulin to the HCl
Vortex
Add insulin dissolved in HCL 0.2 M dropwise to the Phosphate buffer and adjust to pH 7.1-7.3

*Premix preparation*
Prepare 1: 19 parts insulin to WPI slurry and disperse using an Ultraturrax or stomacher. Allow the slurry to sit at 30DegC for 25 minutes. Repeat the Ultraturrax step. Sonicate for 60 seconds. Agitate the slurry at 450 rom at 30DegC for 15minutes.

*Microbead generation*

Use the 150 um or 300 um nozzle.
Use Citrate buffer 0.8 M pH 4.4 (addition of Tween 20 - 0.002%) and heat to 35DegC. Ratio of premix to citrate buffer is specified to generated micro-beads of a specific size (150 - 450 um)
Apply a mild agitation at 150rpm
Maintain Premix under slight stirring during the process (100 rpm).
Adjust the pressure to generate a suitable bead chain.
Frequency and electrode will depend on the viscosity of the premix, time after denaturation, salts utiliised
Frequency: 900-1500 Hz
Electrode 2000-40000 V
Control the process and clean the nozzle/tubing if blocking occurs.
Once the beads are made, leaving sitting in the citrate buffer for 15 min.

*Washing of Wet beads*

Sieve and wash beads through 500 um sieve.
Wash beads in de-ionised water

*Drying - Vacuum*

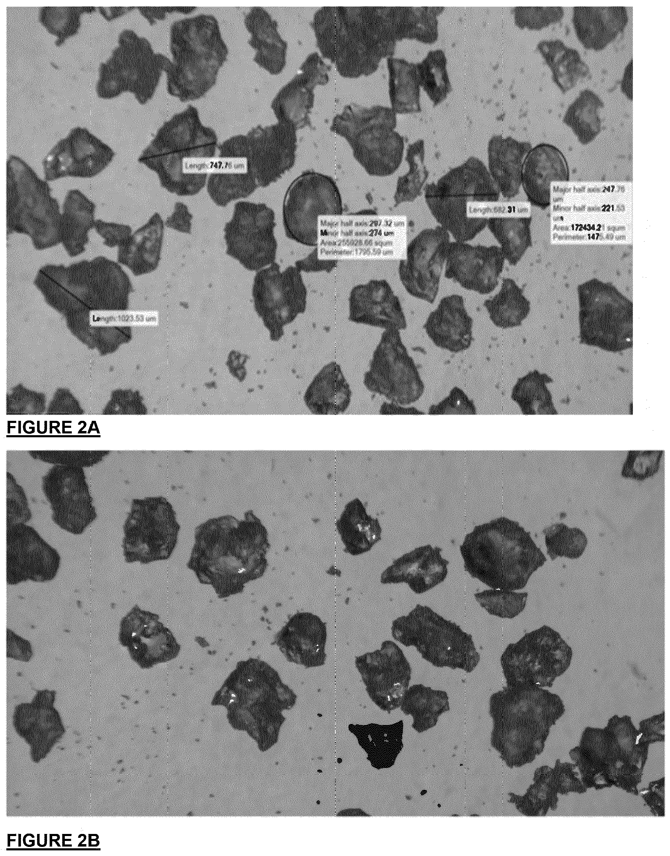

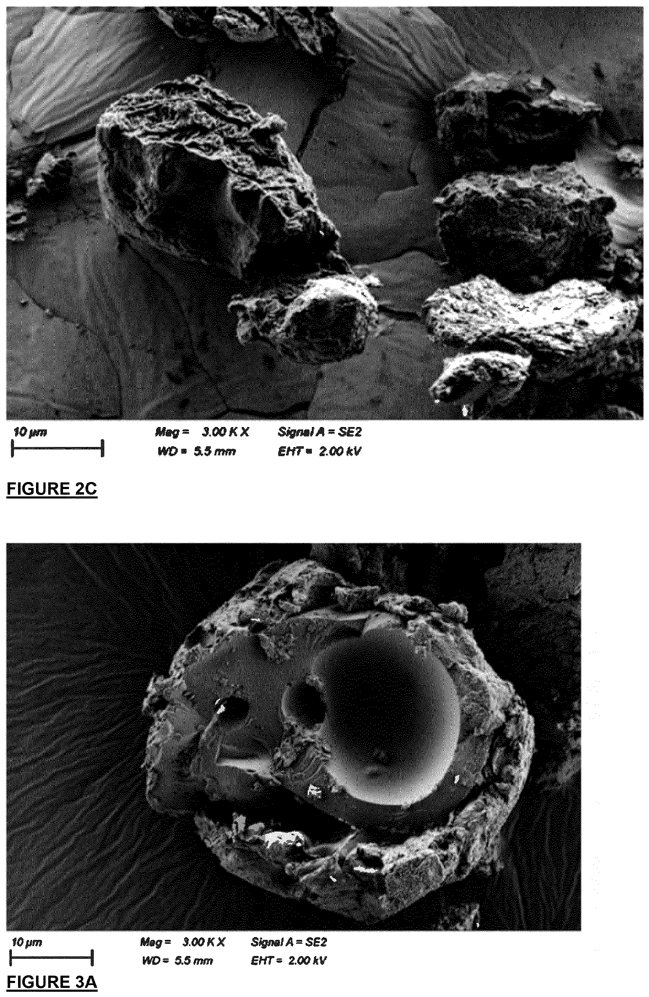

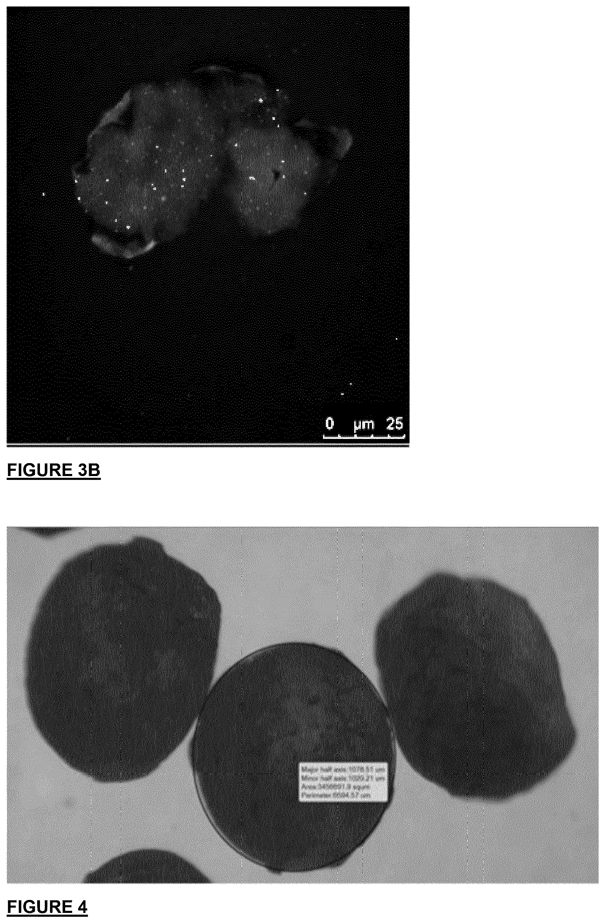

Transfer beads to vacuum air chamber.
Dry for 30 Min - 12 Hour, depending on the bead size and adjust temperature and vacuum pressure accordingly.
Agglomerates of microcapsules formed are shown in Figs 2 and 3. They have an average particle size of 300 - 500 microns.

*Drying Non-vacuum*

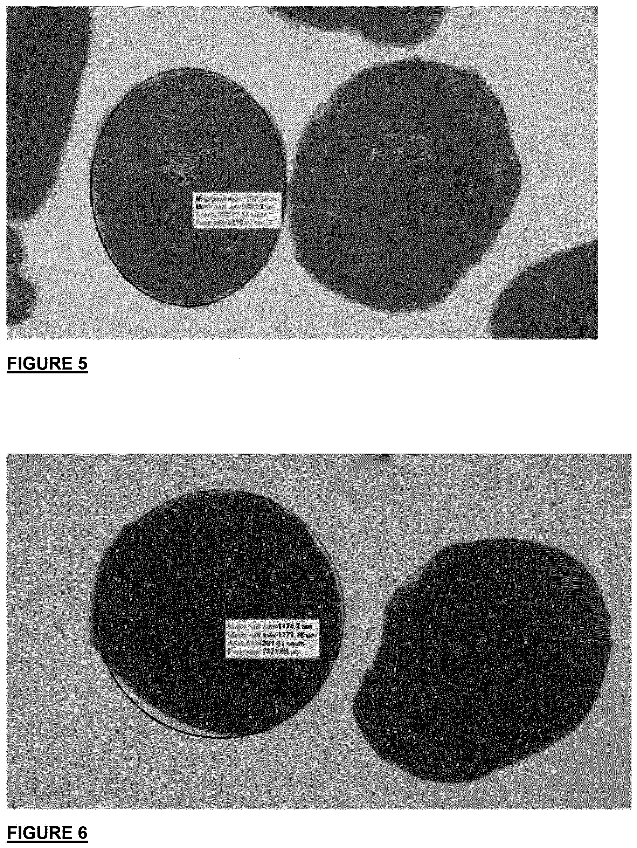

Transfer beads to fluidisd air chamber.
Dry for 1h - 12Hours, depending on the bead size and adjust flow rate from 0.3-25 units per hour.
Agglomerates of microcapsules formed are shown in Figs 4, 5 and 6. They have an average particle size of 1-1.2 mm.

*Sieving and Micro-capsule Charactersation*

Once the drying is finalized, sieve the Micro-capsule obtained

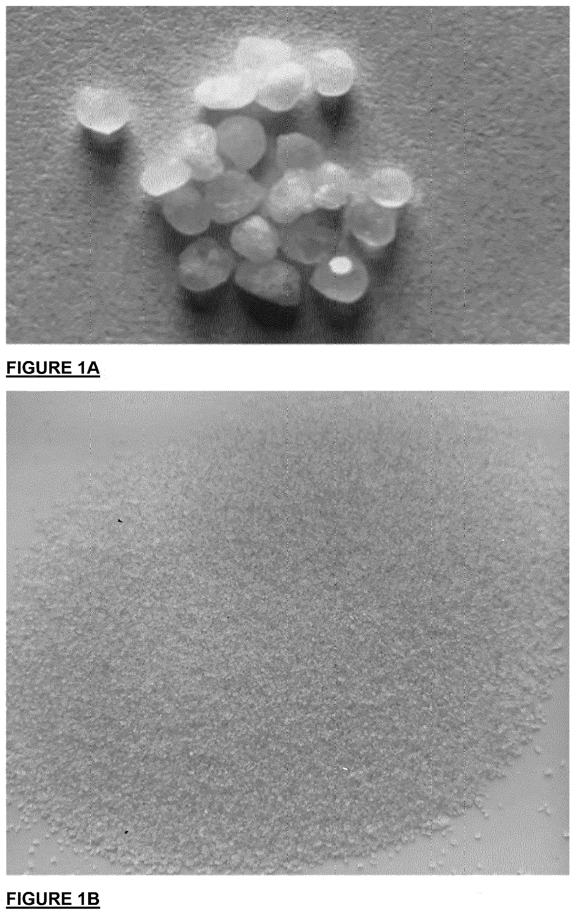

Separate Micro-capsules in the different particle size obtained: > 500 um, 300-500 um, < 300 um. Figure 1 illustrates dried Micro-capsules.

Characterize the required particle size fraction: determine moisture content, quantify insulin using ELISA. Keep the Micro-capsules in a sealed foil bag under dry conditions

## Example 2 - In-vivo study

Microcapsules formed according to Example 1 were administered to a Type I diabetic male human.

An *in vivo* I (acute) study was conducted to evaluate the effect of micro-encapsulated insulin on glucose tolerance and sensitivity in a healthy diabetic (Type 1) and to determine if micro-encapsulated insulin increases physiological uptake of glucose in skeletal muscle of diabetic. Objectives include:

- Objective 1- Identify the concentration of peripherally administered micro-encapsulated insulin that increases glucose clearance following an oral glucose load in Type 1 diabetic human

- Objective 2- Identify whether changes in skeletal muscle glucose uptake are implicated buy micro-encapsulated insulin induced enhancement of glucose clearance in in Type 1 diabetic human.

Microcapsules formed according to Example 1 were administered to a Type I diabetic male human.

The microcapsules were administered at a dosage of from 17.9 IU aspart insulin (0.1 g agglomerated micro-capsules) to 50 IU aspart insulin (0.2 g of agglomerated microcapsules). The composition was administered mid-morning, around 3-4 hours after the morning sub-cutaneous injection when the subject's glucose levels were stable.

The patient's blood glucose was measured immediately prior to administration and 3 hours after administration. The subject's blood glucose levels were continuously measured with Free Style Libre Senor System. The intervention was finished between 3-4 hours after the intake of the micro-capsules.

A subcutaneous injection of 5 IU aspart insulin (FIASP - Novo Nordisk) was employed as a positive control.

Blank microcapsules containing no insulin was used as a negative control.

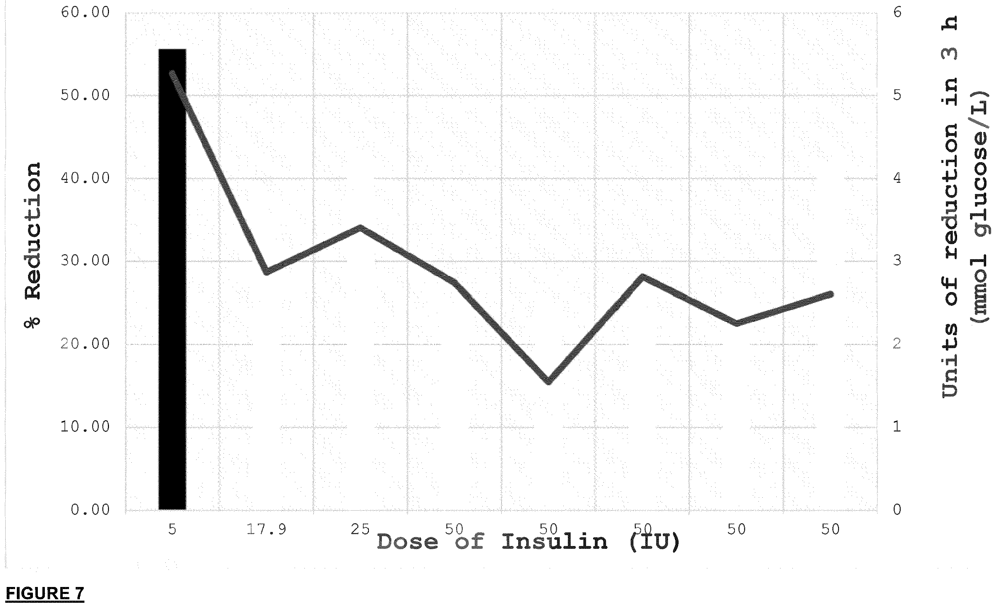

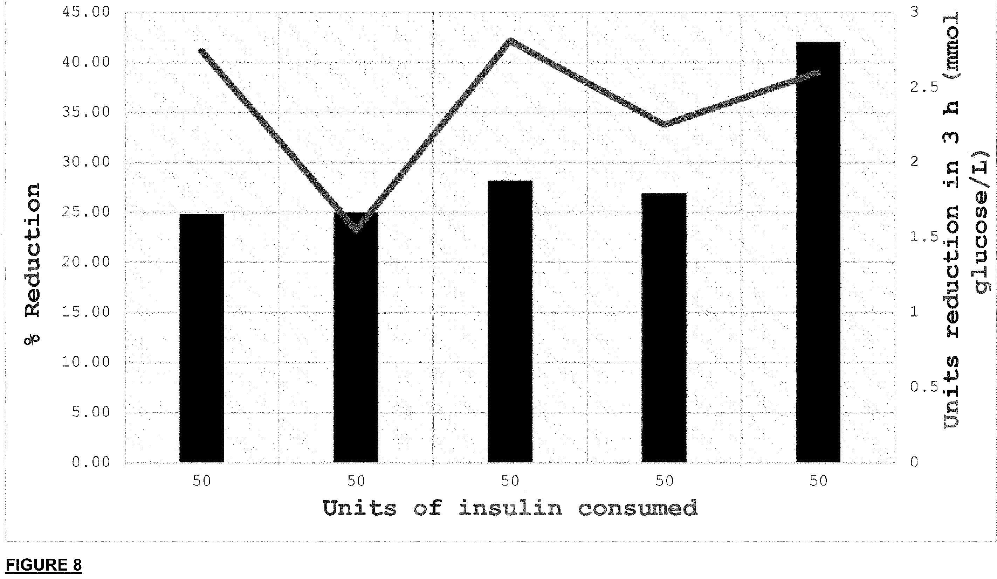

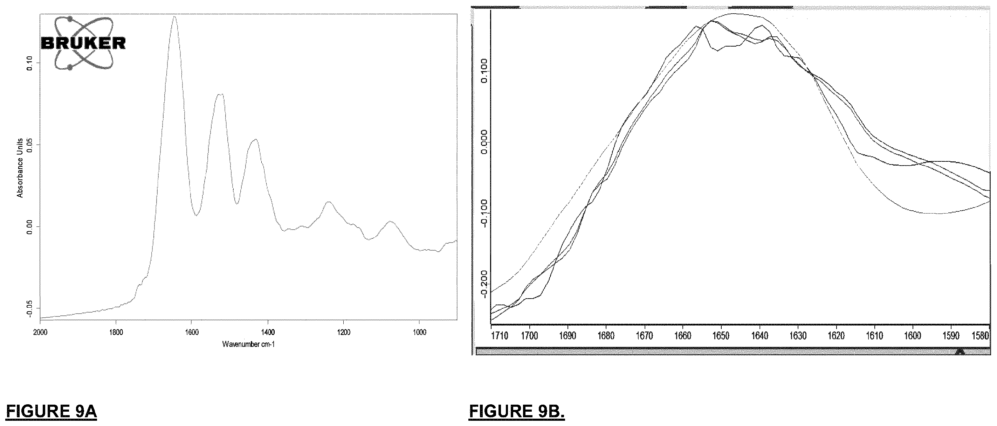

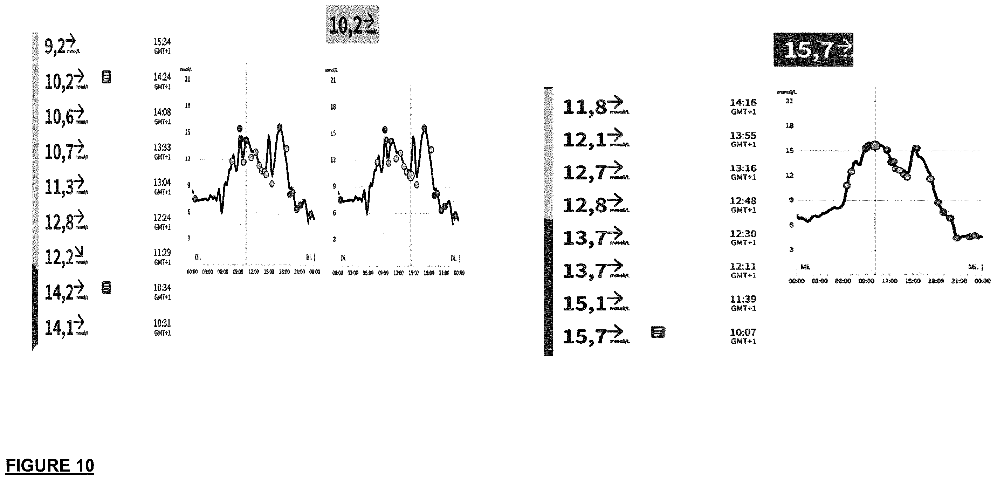

Figures 7 and 8 and 9 illustrate that the composition of the invention reduced blood glucose levels in the patient by 25 - 42% within 3 hours of administration. This compares favourably with the positive control that reduced blood glucose in the same patient by 55% over the same time period. The negative control did not result in a reduction in blood glucose levels over the 3-hour period. Difference in the area under the curve of glucose concentration during injected vs micro-encapsulated insulin were recorded as shown in Figure 10.

## Example 3 - In-vivo study

Microcapsules formed according to Example 1 were administered to a Type I diabetic male human.

An *in vivo* acute study was conducted to evaluate the effect of micro-encapsulated insulin on glucose tolerance and sensitivity in a healthy diabetic (Type 1) and to determine if micro-encapsulated insulin increases physiological uptake of glucose in skeletal muscle of diabetic. Objectives include:

- Objective 1- Identify the concentration of peripherally administered micro-encapsulated insulin that increases glucose clearance following an oral glucose load in Type 1 diabetic human

- Objective 2- Identify whether changes in skeletal muscle glucose uptake are implicated buy micro-encapsulated insulin induced enhancement of glucose clearance in in Type 1 diabetic human.

Microcapsules formed according to Example 1 were administered to a Type I diabetic male human. The microcapsules were administered at a dosage of from 50 IU Human recombinant Insulin (0.05 g agglomerated micro-capsules) to 100 IU Human recombinant insulin (0.1 g of agglomerated microcapsules). The composition was administered mid-morning when the subjects glucose levels were stable.

The patient's blood glucose was measured immediately prior to administration and 3 hours after administration using Free Style Libre Senor System.

A subcutaneous injection of 5 IU aspart insulin (FIASP - Novo Nordisk) was employed as a positive control.

Blank microcapsules containing no insulin was used as a negative control.

The composition of the invention reduced blood glucose levels in the patient by 20 - 50% within 4 hours of administration. This compares favourably with the positive control that reduced blood glucose in the same patient over the same time period.

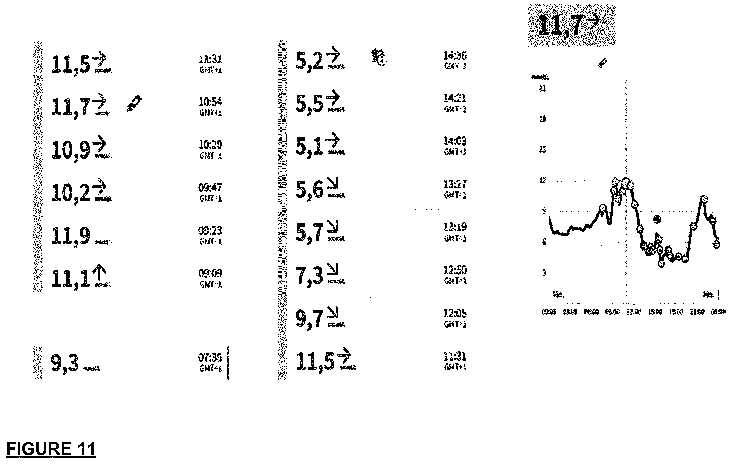

The negative control did not result in a reduction in blood glucose levels over the 3-hour period. Difference in the area under the curve of glucose concentration during injected vs micro-encapsulated insulin were recorded as shown in Figure 11.

## Example 4 - Insulin microcapsules, single stage drying

- Hydrate Whey Protein powder (10.5% - 11% protein content) in water

    ◦ Hydrate and measure pH

- Denature protein at 92 DegC for 15 seconds

    ◦ Cool to room temperature to 22 DegC for 18hours
    ◦ Measure level of protein agglomeration by HPLC

- Hydrate insulin in 0.1N HCl at Room Temperature for 30 minutes to hydrate
- Once hydrated, add 1N NaoH to bring to pH 7.2 and bring volume to 50ml using phosphate saline solution
- Hydrate for 45 minutes at Room Temperature
- Add insulin dropwise to denatured protein (1:5 or 1:20 or 1:50)
- Extrude solution through a single or double nozzle

    ◦ If double nozzle is used, out nozzle is pure denature protein (10% solids)

- Polymerise microcapsules in sodium citrate buffer Ph 4.4 - 4.7. at 30DegC
- Allow solution to polymerise for 2.5 hours at RT in citrate buffer
- Wash insulin micro-capsules in water
- Dry the material under vacuum at room temperature for 18 - 24 hours
- Remove material

    ◦ Measure moisture (<7-8% and Aw content (<0.25)
    ◦ Quantify insulin on HPLC as per usual method

- Store at refrigerated temperature

## Example 5 - Insulin microcapsules, double stage drying

- Hydrate Whey Protein powder ( 10.5% - 11% protein content) in water Hydrate and measure pH
- Denature protein at 92 DegC for 15 seconds 10% - 12.5% protein content)
- Cool to room temperature to 22 DegC for 18hours Measure level of protein agglomeration by HPLC
- Hydrate insulin in 0.1N HCl at RT for 30 minutes to hydrate

Once hydrated, add 1N NaoH to bring to pH 7.2 and bring volume to 50ml using phosphate saline solution

- Hydrate for 45 minutes at Room Temperature
- Add insulin dropwise to denatured protein (1:5 or 1:20 or 1:50) Solids content from 11% - 18% in solution
- Extrude solution through a single or double nozzle
- Polymerise microcapsules in sodium citrate buffer Ph4.4 - 4.7. at 30DegC Allow solution to polymerise for 2.5 hours at Room Temperature in citrate buffer
- Wash insulin micro-capsules in water
- Dry the material under vacuum at room temperature for 18 - 24 hours at <15 mBar with agitation

∘ Remove material and measure moisture (<7-8% and Aw content (<0.25)

- Transfer to secondary chamber for secondary drying for further 24-36 hour drying at <10 mBar (2nd stage drying) with / without agitation
- Remove material and pack

∘ Measure moisture (<5% and Aw content (<0.2) of finsihed product
∘ Quantify insulin on HPLC as per usual method

- Store at refrigerated temperature

**Claims**

1. A pharmaceutical composition in an oral dosage form comprising gastric-resistant ileal-sensitive microcapsules comprising a matrix and insulin contained within the matrix, in which the matrix comprises denatured whey protein, in which the microcapsules are cold-gelated and vacuum dried microcapsules, in which the matrix of the microcapsules is free of polysaccharide gelling agent.

2. A pharmaceutical composition of Claim 1, in which at least 90% of the microcapsules in the preparation are capable of surviving intact in fresh ex-vivo porcine gastric juice of pH 2.0 for at least three hours at 37°C under agitation at 150rpm.

3. A pharmaceutical composition of Claim 1 or 2, in which the microcapsules comprise as a dry weight %:

   90 to 99 % denatured whey protein; and
   1 to 10 % insulin.

4. A pharmaceutical composition of any of Claims 1 to 3, in which the matrix consists essentially of denatured whey protein.

5. A pharmaceutical composition of any of Claims 1 to 4, in which the microcapsules have a water activity (Aw) of less than 0.25.

6. A pharmaceutical composition of any of Claims 1 to 5, in which the microcapsules have a water activity (Aw) of 0.20 or less.

7. A pharmaceutical composition of any of Claims 1 to 6, in which the oral dosage form is a tablet formed by compression of the microcapsules.

8. A pharmaceutical composition of any of Claims 1 to 6, in which the oral dosage form is a tablet formed by direct compression of the microcapsules.

9. A method of making microcapsules comprising the steps of

   providing microdroplets comprising denatured whey protein and insulin by extrusion;
   curing the microdroplets by immersion in a curing bath to form microcapsules having a matrix comprising denatured whey protein and insulin contained within the matrix;
   removing the microcapsules from the curing bath; and
   vacuum drying the microcapsules,
   in which the matrix of the microcapsules is free of polysaccharide gelling agent.

10. A method according to Claim 9, in which the microdroplets are generated (a) using concentric nozzles by extruding a suspension or solution of the insulin and denatured whey protein through an inner nozzle and simultaneously extruding a denatured whey protein suspension through an outer nozzle, or (b) by extruding a suspension comprising denatured whey protein and insulin through a single nozzle.

11. A method according to Claim 9 or 10, in which the suspension comprising denatured whey protein comprises 10-20% denatured whey protein.

12. A method according to any of Claims 9 to 11, in which the step of providing the microdroplets comprises the steps of:

> dissolving insulin in an acid to provide an insulin solution;
> combining the insulin solution with a buffer to provide a buffered insulin solution with a pH to 6-8; and
> combining the buffered insulin solution with the suspension comprising denatured whey protein prior to the extrusion step.

13. A method according to Claim 12, in which the buffered insulin solution is added to the suspension comprising denatured whey protein dropwise.

14. A method according to any of Claims 9 to 13, in which the microcapsules are subjected to a first vacuum drying step and a second vacuum drying step.

15. A method according to any of Claims 9 to 14, in which: the microdroplets consist essentially of denatured whey protein, insulin and solvent, and/or in which the microcapsules prior to drying have a water activity (Aw) of 0.993 - 0.997.

## Patentansprüche

1. Pharmazeutische Zusammensetzung in einer oralen Dosierungsform, die magenbeständige, Ileum-empfindliche Mikrokapseln umfasst, die eine Matrix und Insulin, das innerhalb der Matrix enthalten ist, umfassen, wobei die Matrix denaturiertes Molkenprotein umfasst, wobei die Mikrokapseln kalt gelierte und vakuumgetrocknete Mikrokapseln sind, wobei die Matrix der Mikrokapseln frei von Polysaccharid-Geliermittel ist.

2. Pharmazeutische Zusammensetzung nach Anspruch 1, wobei mindestens 90 % der Mikrokapseln in dem Präparat unbeschädigt in frischem ex-vivo Magensaft vom Schwein von pH 2,0 für mindestens drei Stunden bei 37 °C unter Rühren bei 150 U/min überdauern können.

3. Pharmazeutische Zusammensetzung nach Anspruch 1 oder 2, wobei die Mikrokapseln Trockengewicht-% von Folgendem umfassen:

> 90 bis 99 % denaturiertes Molkenprotein; und
> 1 bis 10 % Insulin.

4. Pharmazeutische Zusammensetzung nach einem der Ansprüche 1 bis 3, wobei die Matrix im Wesentlichen aus denaturiertem Molkenprotein besteht.

5. Pharmazeutische Zusammensetzung nach einem der Ansprüche 1 bis 4, wobei die Mikrokapseln eine Wasseraktivität (Aw) von weniger als 0,25 aufweisen.

6. Pharmazeutische Zusammensetzung nach einem der Ansprüche 1 bis 5, wobei die Mikrokapseln eine Wasseraktivität (Aw) von 0,20 oder weniger aufweisen.

7. Pharmazeutische Zusammensetzung nach einem der Ansprüche 1 bis 6, wobei die orale Dosierungsform eine Tablette ist, die durch Kompression der Mikrokapseln gebildet wird.

8. Pharmazeutische Zusammensetzung nach einem der Ansprüche 1 bis 6, wobei die orale Dosierungsform eine Tablette ist, die durch direkte Kompression der Mikrokapseln gebildet wird.

9. Verfahren zur Herstellung von Mikrokapseln, das die folgenden Schritte umfasst:

> Bereitstellen von Mikrotröpfchen, die denaturiertes Molkenprotein und Insulin umfassen, durch Extrusion;
> Aushärten der Mikrotröpfchen durch Eintauchen in ein Härtungsbad, um Mikrokapseln zu bilden, die eine Matrix, die denaturiertes Molkenprotein umfasst, und Insulin, das innerhalb der Matrix enthalten ist, aufweisen;
> Entnehmen der Mikrokapseln aus dem Härtungsbad; und
> Vakuumtrocknen der Mikrokapseln,
> wobei die Matrix der Mikrokapseln frei von Polysaccharid-Geliermittel ist.

10. Verfahren nach Anspruch 9, wobei die Mikrotröpfchen durch Folgendes erzeugt werden: (a) unter Verwendung von konzentrischen Düsen durch Extrudieren einer Suspension oder Lösung des Insulins und denaturierten Molkenproteins durch eine innere Düse und gleichzeitiges Extrudieren einer Suspension des denaturierten Molkenproteins durch eine äußere Düse oder (b) durch Extrudieren einer Suspension, die denaturiertes Molkenprotein und Insulin umfasst, durch eine einzige Düse.

11. Verfahren nach Anspruch 9 oder 10, wobei die Suspension, die denaturiertes Molkenprotein umfasst, 10-20 % denaturiertes Molkenprotein umfasst.

12. Verfahren nach einem der Ansprüche 9 bis 11, wobei der Schritt des Bereitstellens der Mikrotröpfchen die folgenden Schritte umfasst:

Lösen von Insulin in einer Säure, um eine Insulinlösung bereitzustellen;
Vereinigen der Insulinlösung mit einem Puffer, um eine gepufferte Insulinlösung mit einem pH bis 6-8 bereitzustellen; und
Vereinigen der gepufferten Insulinlösung mit der Suspension, die denaturiertes Molkenprotein umfasst, vor dem Extrusionsschritt.

13. Verfahren nach Anspruch 12, wobei die gepufferte Insulinlösung zu der Suspension, die denaturiertes Molkenprotein umfasst, tropfenweise zugegeben wird.

14. Verfahren nach einem der Ansprüche 9 bis 13, wobei die Mikrokapseln einem ersten Vakuumtrocknungsschritt und einem zweiten Vakuumtrocknungsschritt unterzogen werden.

15. Verfahren nach einem der Ansprüche 9 bis 14, wobei die Mikrotröpfchen im Wesentlichen aus denaturiertem Molkenprotein, Insulin und Lösungsmittel bestehen und/oder wobei die Mikrokapseln vor dem Trocknen eine Wasseraktivität (Aw) von 0,993-0,997 aufweisen.

**Revendications**

1. Composition pharmaceutique sous forme de dosage oral comprenant des microcapsules résistantes à l'estomac et sensibles à l'iléon, comprenant une matrice et de l'insuline contenue au sein de la matrice, dans laquelle la matrice comprend des protéines de lactosérum dénaturées, où les microcapsules sont des microcapsules gélifiées à froid et séchées sous vide, où la matrice des microcapsules est exempte d'agent gélifiant polysaccharidique.

2. Composition pharmaceutique selon la revendication 1, dans laquelle au moins 90% des microcapsules de la préparation sont capables de rester intactes dans du suc gastrique porcin *ex vivo* frais de pH 2,0 pendant au moins trois heures à 37°C sous agitation à 150 tours/min.

3. Composition pharmaceutique selon la revendication 1 ou 2, dans laquelle les microcapsules comprennent, en pourcentage de poids sec :

de 90 à 99% de protéines de lactosérum dénaturées ; et
de 1 à 10% d'insuline.

4. Composition pharmaceutique selon l'une quelconque des revendications 1 à 3, dans laquelle la matrice est constituée essentiellement de protéines de lactosérum dénaturées.

5. Composition pharmaceutique selon l'une quelconque des revendications 1 à 4, dans laquelle les microcapsules ont une activité de l'eau (Aw) inférieure à 0,25.

6. Composition pharmaceutique selon l'une quelconque des revendications 1 à 5, dans laquelle les microcapsules ont une activité de l'eau (Aw) de 0,20 ou moins.

7. Composition pharmaceutique selon l'une quelconque des revendications 1 à 6, dans laquelle la forme de dosage oral est un comprimé formé par compression des microcapsules.

8. Composition pharmaceutique selon l'une quelconque des revendications 1 à 6, dans laquelle la forme de dosage oral est un comprimé formé par compression directe des microcapsules.

9. Méthode de fabrication de microcapsules, comprenant les étapes :

de mise à disposition de microgouttelettes comprenant des protéines de lactosérum dénaturées et de l'insuline par extrusion ;
de durcissement des microgouttelettes par immersion dans un bain de durcissement pour former des microcapsules ayant une matrice comprenant des protéines de lactosérum dénaturées et de l'insuline contenue au sein de la matrice ;
de retrait des microcapsules du bain de durcissement ; et
de séchage sous vide des microcapsules,

dans laquelle la matrice des microcapsules est exempte d'agent gélifiant polysaccharidique.

10. Méthode selon la revendication 9, dans laquelle les microgouttelettes sont générées (a) à l'aide de buses concentriques en extrudant une suspension ou une solution d'insuline et de protéines de lactosérum dénaturées à travers une buse interne et en extrudant simultanément une suspension de protéines de lactosérum dénaturées à travers une buse externe, ou (b) en extrudant une suspension comprenant des protéines de lactosérum dénaturées et de l'insuline à travers une seule buse.

11. Méthode selon la revendication 9 ou 10, dans laquelle la suspension comprenant des protéines de lactosérum dénaturées comprend 10-20% de protéines de lactosérum dénaturées.

12. Méthode selon l'une quelconque des revendications 9 à 11, dans laquelle l'étape de mise à disposition des microgouttelettes comprend les étapes :

de solubilisation d'insuline dans un acide pour obtenir une solution d'insuline ;
de combinaison de la solution d'insuline avec un tampon pour obtenir une solution d'insuline tamponnée ayant un pH de 6-8 ; et
de combinaison de la solution d'insuline tamponnée avec la suspension comprenant des protéines de lactosérum dénaturées préalablement à l'étape d'extrusion.

13. Méthode selon la revendication 12, dans laquelle la solution d'insuline tamponnée est ajoutée goutte à goutte à la suspension comprenant des protéines de lactosérum dénaturées.

14. Méthode selon l'une quelconque des revendications 9 à 13, dans laquelle les microcapsules sont soumises à une première étape de séchage sous vide et à une deuxième étape de séchage sous vide.

15. Méthode selon l'une quelconque des revendications 9 à 14, dans laquelle les microgouttelettes sont constituées essentiellement de protéines de lactosérum dénaturées, d'insuline et de solvant, et/ou dans laquelle les microcapsules, avant séchage, ont une activité de l'eau (Aw) de 0,993-0,997.

**FIGURE 1A**

**FIGURE 1B**

**FIGURE 2A**

**FIGURE 2B**

10 µm    Mag = 3.00 K X    Signal A = SE2
         WD = 5.5 mm       EHT = 2.00 kV

**FIGURE 2C**

10 µm    Mag = 3.00 K X    Signal A = SE2
         WD = 5.5 mm       EHT = 2.00 kV

**FIGURE 3A**

FIGURE 3B

FIGURE 4

**FIGURE 5**

**FIGURE 6**

**FIGURE 7**

**FIGURE 8**

EP 4 188 328 B1

**FIGURE 9B.**

**FIGURE 9A**

23

FIGURE 10

FIGURE 11

25

FIG.12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010119041 A
- WO 2014198787 A
- WO 2016096929 A

**Non-patent literature cited in the description**

- **DEAT-LAINE et al.** *International Journal of Pharmaceutics*, 2012, vol. 239
- *Pharmaceutical Research*, vol. 30, 20212
- *International Journal of Pharmaceutics*, 2013, vol. 453
- **EMMANUELLE DÉAT-LAINÉ et al.** Efficacy of Mucoadhesive Hydrogel Microparticles of Whey Protein and Alginate for Oral Insulin Delivery. *PHARMACEUTICAL RESEARCH*, 24 October 2012, vol. 30 (3), 721-734
- **S.B. DOHERTY** ; **V.L. GEE** ; **R.P. ROSS** ; **C. STANTON** ; **G.F. FITZGERALD** ; **A. BRODKORB**. *Food Hydrocolloids*, August 2011, vol. 25 (6), 1604-1617
- **MINEKUS, M.** ; **SMEETS-PEETERS M** ; **BERNALIER A** ; **MAROL-BONNIN S** ; **HAVENAAR R** ; **MARTEAU P** ; **ALRIC M** ; **FONTY G** ; **HUIS IN'T VELD JH**. A computer-controlled system to simulate conditions of the large intestine with peristaltic mixing, water absorption and absorption of fermentation product. *Applied Microbiology Biotechnology*, December 1999, vol. 53 (1), 108-14
- **MINEKUS et al.** *A standardised static in vitro digestion method suitable for food - an international consensus*, 2014
- **MINEKUS, A. et al.** *Food Function*, 2014, vol. 5, 1113
- **CARTER, B. P.** ; **GALLOWAY, M. T.** ; **CAMPBELL, G. S.** ; **CARTER, A. H.** The critical water activity from dynamic dewpoint isotherms as an indicator of premix powder stability. *Journal of Food Measurement and Characterization*, 2015, vol. 9 (4), 479-486